# EUROPEAN PATENT SPECIFICATION

(11) **EP 2 895 160 B1**
(45) Date of publication and mention of the grant of the patent: **30.05.2018**
(21) Application number: 13762485.4
(22) Date of filing: 13.09.2013
(51) Int. Cl.: A61K 31/196, A61K 8/42, A61Q 7/00, A61K 45/06, A61P 17/14

(54) **METHODS OF TREATING HAIR RELATED CONDITIONS**
VERFAHREN ZUR BEHANDLUNG VON ERKRANKUNGEN IM ZUSAMMENHANG MIT HAAR
MÉTHODES DE TRAITEMENT DE TROUBLES LIÉS AUX CHEVEUX

(30) Priority: 13.09.2012 US 201261700623 P
(43) Date of publication of application: 22.07.2015
(73) Proprietor: Nogra Pharma Limited, Dublin 2 (IE)
(72) Inventor: GIULIANI, Giammaria, 20123 Milan (IT); PAUS, Ralf, 22339 Hamburg (DE); RAMOT, Yuval, 90805 Mevasseret Zion (IL); BARONI, Sergio, 24030 Villa D'adda (IT); VITI, Francesca, 6872 Salorino (CH); BELLINVIA, Salvatore, 6850 Mendrisio (CH); MARZANI, Barbara, 27020 Carbonara Al Ticino (IT)
(74) Representative: Tomkins & Co
(86) International application number: PCT/EP2013/069062
(87) International publication number: WO 2014/041140

(56) References cited:
- WO-A2-2010/091892
- WO-A2-2010/091894
- Paradi Mirmirani: "Lichen Planopilaris Treated With a Peroxisome Proliferator-Activated Receptor [gamma] Agonist", Archives of Dermatology, vol. 145, no. 12, 1 December 2009 (2009-12-01), page 1363, XP055366047, US ISSN: 0003-987X, DOI: 10.1001/archdermatol.2009.283

## Description

### BACKGROUND

Skin and its appendix hair follicle structure have a well-organized architecture and an excellent system characterized by molecular mechanisms that regulate stem cell self-renewal, proliferation, migration and lineage commitment. Each hair follicle is composed of a permanent portion, which includes sebaceous glands and the underlying bulge area, and a dynamic renewing portion, which undergo cycles of anagen (an active growth phase), catagen (a remodeling phase) and finally telogen (a quiescent phase). Two key elements that control hair follicle cycling are the follicular epithelial stem cells, located in the bulge area of the hair follicle, and the specialized mesenchymal cells that constitute the follicular papilla.

Epithelial stem cells are multipotent, giving rise to daughter cells that either migrate upward to serve as epidermal progenitors for generating epidermal cells during wound repair or migrate downward to convert to hair-matrix progenitors, which further give rise to the hair shaft.

There are several reasons why hair growth can slow or cease. The production of hair fibers can cease, for example, because the matrix cells have exhausted their proliferative capacities: this assumes that the proliferative capacity of matrix cells is determined once for all at the initiation of a new hair cycle and that new matrix cells are not generated for the entire growth phase.

Stem cells may continuously generate new matrix cells. Production of hair fibers can cease when the stem cells are instructed to stop generating new progeny.

Some types of alopecia, classified as cicatricial or scarring alopecia, such as lichen planopilaris, frontal fibrosing alopecia, chronic cutaneous lupus erythematosus, keratosis follicularis, spinulosa decalvans or folliculitis decalvans, are disorders that cause destruction of hair follicle stem cells in the bulge area and permanent hair loss. Thus, preventing destruction of hair follicle stem cells so that a hair follicle's basic capacity to regenerate itself is retained may permit the regrowth of hair in patients suffering from disorders related to stem cell destruction.

Mirimani et al. (Arch Dermatol. 2009, 145(12), 1363) describes a case strudy where an individual diagnosed with lichen planopilaris received pioglitazone hydrochloride orally and employed an antiseborrheic shampoo. Persistent alopecia with loss of follicular hair markings was observed.

Accordingly, there is a need for effective agents that preserve hair follicle stem cells such that the hair follicle is retained and capable of regeneration.

### SUMMARY

In one aspect the present invention provides a pharmaceutical composition comprising N-acetyl-3-(4'-aminophenyl)-2-methoxypropionic acid, or a pharmaceutically acceptable salt or a stereoisomer thereof, and a pharmaceutically acceptable excipient, for use in treating or ameliorating lichen planopilaris in a patient in need thereof, wherein the composition is for topical administration to said patient, as set out in the claims.

The disclosure provides for a pharmaceutical composition for use in treating or ameliorating lichen planopilaris as set out in the claims, which enhances epidermal regeneration in a patient in need thereof. The pharmaceutically acceptable composition comprising a compound disclosed herein (e.g., N-acetyl- 3-(4-aminophenyl)-2-methoxypropionic acid) or a pharmaceutically acceptable salt or stereoisomer thereof, and a pharmaceutically acceptable excipient is for topical administration.

Disclosed herein are methods of treating, or ameliorating cicatricial alopecia, comprising topically administering to a patient in need thereof a pharmaceutically acceptable composition comprising a compound disclosed herein. For example, provided herein is a method of treating, or ameliorating cicatricial alopecia, comprising topically administering to a patient in need thereof a pharmaceutically acceptable composition comprising N-acetyl-3-(4-aminophenyl)-2-methoxypropionic acid or a pharmaceutically acceptable salt or stereoisomer thereof, and a pharmaceutically acceptable excipient.

Also disclosed herein are methods for protecting hair follicle progenitor cells before depilation of mammalian skin, comprising contacting said cells with a pharmaceutically acceptable composition comprising a compound disclosed herein (e.g., N-acetyl-3-(4-aminophenyl)-2-methoxypropionic acid) or a pharmaceutically acceptable salt or stereoisomer thereof.

Disclosed herein is a method for protecting and/or preserving a composition comprising hair follicle progenitor cells, wherein the method comprises contacting said hair follicle progenitor cells with N-acetyl-3-(4-aminophenyl)-2-methoxypropionic acid is also provided.

Also disclosed herein are methods of promoting wound healing in a patient, comprising administering to said patient a pharmaceutically acceptable composition comprising a compound disclosed herein (e.g., N-acetyl-3-(4-aminophenyl)-2-methoxypropionic acid) or a pharmaceutically acceptable salt or stereoisomer thereof, and a pharmaceutically acceptable excipient.

### BRIEF DESCRIPTION OF THE FIGURES

Figure 1 depicts the experimental design of the study described in Example 2.
Figure 2 depicts pooled data showing stimulation of K-15-immunoreactivity following administration of N-acetyl-(R)-(-)-3-(4-aminophenyl)-2-methoxypropionic acid.
Figure 3 shows DPAI staining of K15-positive cells.
Figure 4 depicts pooled data showing stimulation of K-19-immunoreactivity following administration of N-acetyl-(R)-(-)-3-(4-aminophenyl)-2-methoxypropionic acid.
Figure 5 shows DPAI staining of K19-positive cells.
Figure 6 depicts pooled data showing increased numbers of K19 positive cells in the low and high concentrations after administration of N-acetyl-(R)-(-)-3-(4-aminophenyl)-2-methoxypropionic acid.
Figure 7 depicts the increase in LDH activity after administration of N-acetyl-(R)-(-)-3-(4-aminophenyl)-2-methoxypropionic acid in the 0.1 mM dose on day 6 (pooled data).
Figure 8 depicts inhibition of hair shaft elongation after administration of high dose (1 mM) N-acetyl-(R)-(-)-3-(4-aminophenyl)-2-methoxypropionic acid on day 6 (pooled data).
Figure 9 depicts N-acetyl-(R)-(-)-3-(4-aminophenyl)-2-methoxypropionic acid catagen at various concentrations (pooled data).
Figure 10 depicts trend toward catagen at various dosages of N-acetyl-(R)-(-)-3-(4-aminophenyl)-2-methoxypropionic acid (pooled data, comparing the percentage of each hair cycle stage in each experiment, n=5; 0.4 = 40%)

### DETAILED DESCRIPTION

The features and other details of the disclosure will now be more particularly described. Before further description of the present invention, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

### Definitions

"Treating" includes any effect, e.g., lessening, reducing, modulating, or eliminating, that results in the improvement of the condition, disease, disorder and the like.

The term "alkenyl" as used herein refers to an unsaturated straight or branched hydrocarbon having at least one carbon-carbon double bond, such as a straight or branched group of 2-12, 2-10, or 2-6 carbon atoms, referred to herein as C₂-C₁₂alkenyl, C₂-C₁₀alkenyl, and C₂-C₆alkenyl, respectively. Exemplary alkenyl groups include, but are not limited to, vinyl, allyl, butenyl, pentenyl, hexenyl, butadienyl, pentadienyl, hexadienyl, 2-ethylhexenyl, 2-propyl-2-butenyl, 4-(2-methyl-3-butene)-pentenyl, etc.

The term "alkoxy" as used herein refers to an alkyl group attached to an oxygen (-O-alkyl-). Exemplary alkoxy groups include, but are not limited to, groups with an alkyl, alkenyl or alkynyl group of 1-12, 1-8, or 1-6 carbon atoms, referred to herein as C₁-C₁₂alkoxy, C₁-C₈alkoxy, and C₁-C₆alkoxy, respectively. Exemplary alkoxy groups include, but are not limited to methoxy, ethoxy, etc. Similarly, exemplary "alkenoxy" groups include, but are not limited to vinyloxy, allyloxy, butenoxy, etc.

The term "alkyl" as used herein refers to a saturated straight or branched hydrocarbon, such as a straight or branched group of 1-12, 1-10, or 1-6 carbon atoms, referred to herein as C₁-C₁₂alkyl, C₁-C₁₀alkyl, and C₁-C₆alkyl, respectively. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, octyl, etc. Alkyl may refer to C₁-C₆ alkyl. Cycloalkyl may refer to C₃-C₆cycloalkyl.

Alkyl, alkenyl and alkynyl groups can be optionally be substituted with or interrupted by at least one group selected from alkanoyl, alkoxy, alkyl, alkenyl, alkynyl, amido, amidino, amino, aryl, arylalkyl, azido, carbamate, carbonate, carboxy, cyano, cycloalkyl, ester, ether, formyl, halogen, haloalkyl, heteroaryl, heterocyclyl, hydroxyl, imino, ketone, nitro, phosphate, phosphonato, phosphinato, sulfate, sulfide, sulfonamido, sulfonyl and thiocarbonyl.

The term "alkynyl" as used herein refers to an unsaturated straight or branched hydrocarbon having at least one carbon-carbon triple bond, such as a straight or branched group of 2-12, 2-8, or 2-6 carbon atoms, referred to herein as C₂-C₁₂alkynyl, C₂-C₈alkynyl, and C₂-C₆alkynyl, respectively. Exemplary alkynyl groups include, but are not limited to, ethynyl, propynyl, butynyl, pentynyl, hexynyl, methylpropynyl, 4-methyl-1-butynyl, 4-propyl-2-pentynyl, and 4-butyl-2-hexynyl, etc.

The term "amide" or "amido" as used herein refers to a radical of the form -RₐC(O)N(R_{b})-, -RₐC(O)N(R_{b})R_{c}-, or -C(O)NR_{b}R_{c}, wherein Rₐ, R_{b} and R_{c} are each independently selected from alkoxy, alkyl, alkenyl, alkynyl, amide, amino, aryl, arylalkyl, carbamate, cycloalkyl, ester, ether, formyl, halogen, haloalkyl, heteroaryl, heterocyclyl, hydrogen, hydroxyl, ketone, and nitro. The amide can be attached to another group through the carbon, the nitrogen, R_{b}, R_{c}, or Rₐ. The amide also may be cyclic, for example R_{b} and R_{c}, Rₐ and R_{b}, or Rₐ and R_{c} may be joined to form a 3- to 12-membered ring, such as a 3- to 10-membered ring or a 5- to 6-membered ring. The term "carboxamido" refers to the structure -C(O)NR_{b}R_{c}.

The term "amine" or "amino" as used herein refers to a radical of the form -NR_{d}Rₑ, -N(R_{d})Rₑ-, or -RₑN(R_{d})R_{f}- where R_{d}, Rₑ, and R_{f} are independently selected from alkoxy, alkyl, alkenyl, alkynyl, amide, amino, aryl, arylalkyl, carbamate, cycloalkyl, ester, ether, formyl, halogen, haloalkyl, heteroaryl, heterocyclyl, hydrogen, hydroxyl, ketone, and nitro. The amino can be attached to the parent molecular group through the nitrogen, R_{d}, Rₑ or R_{f}. The amino also may be cyclic, for example any two of Rd, Re or Rf may be joined together or with the N to form a 3- to 12-membered ring, e.g., morpholino or piperidinyl. The term amino also includes the corresponding quaternary ammonium salt of any amino group, e.g., -[N(Rd)(Re)(Rf)]+. Exemplary amino groups include aminoalkyl groups, wherein at least one of R_{d}, Rₑ, or R_{f} is an alkyl group.

The term "cycloalkoxy" as used herein refers to a cycloalkyl group attached to an oxygen.

The term "cycloalkyl" as used herein refers to a monovalent saturated or unsaturated cyclic, bicyclic, or bridged bicyclic hydrocarbon group of 3-12, 3-8, 4-8, or 4-6 carbons, referred to herein, e.g., as "C₄₋₈cycloalkyl," derived from a cycloalkane. Exemplary cycloalkyl groups include, but are not limited to, cyclohexanes, cyclohexenes, cyclopentanes, cyclopentenes, cyclobutanes and cyclopropanes. Cycloalkyl groups may be substituted with alkanoyl, alkoxy, alkyl, alkenyl, alkynyl, amido, amidino, amino, aryl, arylalkyl, azido, carbamate, carbonate, carboxy, cyano, cycloalkyl, ester, ether, formyl, halogen, haloalkyl, heteroaryl, heterocyclyl, hydroxyl, imino, ketone, nitro, phosphate, phosphonato, phosphinato, sulfate, sulfide, sulfonamido, sulfonyl and thiocarbonyl. Cycloalkyl groups can be fused to other cycloalkyl, aryl, or heterocyclyl groups. Cycloalkyl may refer to C₃-C₆ alkyl.

The terms "halo" or "halogen" or "Hal" as used herein refer to F, Cl, Br, or I.

The term "haloalkyl" as used herein refers to an alkyl group substituted with one or more halogen atoms.

The term "nitro" as used herein refers to the radical -NO₂.

The term "phenyl" as used herein refers to a 6-membered carbocyclic aromatic ring. The phenyl group can also be fused to a cyclohexane or cyclopentane ring. Phenyl can be substituted with one or more substituents including alkanoyl, alkoxy, alkyl, alkenyl, alkynyl, amido, amidino, amino, aryl, arylalkyl, azido, carbamate, carbonate, carboxy, cyano, cycloalkyl, ester, ether, formyl, halogen, haloalkyl, heteroaryl, heterocyclyl, hydroxyl, imino, ketone, nitro, phosphate, phosphonato, phosphinato, sulfate, sulfide, sulfonamido, sulfonyl and thiocarbonyl.

The term "pharmaceutically acceptable carrier" or "pharmaceutically acceptable excipient" as used herein refers to any and all solvents, dispersion media, coatings, isotonic and absorption delaying agents, and the like, that are compatible with pharmaceutical administration. The use of such media and agents for pharmaceutically active substances is well known in the art. The compositions may also contain other active compounds providing supplemental, additional, or enhanced therapeutic functions.

The term "pharmaceutical composition" as used herein refers to a composition comprising at least one compound as disclosed herein formulated together with one or more pharmaceutically acceptable carriers.

"Individual," "patient," or "subject" are used interchangeably and include to any animal, including mammals, preferably mice, rats, other rodents, rabbits, dogs, cats, swine, cattle, sheep, horses, or primates, and most preferably humans. The compounds disclosed herein can be administered to a mammal, such as a human, but can also be other mammals such as an animal in need of veterinary treatment, *e.g*., domestic animals (*e.g*., dogs, cats, and the like), farm animals (*e.g*., cows, sheep, pigs, horses, and the like) and laboratory animals (*e.g*., rats, mice, guinea pigs, and the like). "Modulation" includes antagonism (*e.g*., inhibition), agonism, partial antagonism and/or partial agonism.

In the present specification, the term "therapeutically effective amount" means the amount of the subject compound that will elicit the biological or medical response of a tissue, system, animal or human that is being sought by the researcher, veterinarian, medical doctor or other clinician. The compounds disclosed herein are administered in therapeutically effective amounts to treat a disease. Alternatively, a therapeutically effective amount of a compound is the quantity required to achieve a desired therapeutic and/or prophylactic effect, such as an amount which results in the prevention of or a decrease in the symptoms associated with a disease or disorder.

The term "pharmaceutically acceptable salt(s)" as used herein refers to salts of acidic or basic groups that may be present in compounds used in the present compositions. Compounds included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, i.e., salts containing pharmacologically acceptable anions, including but not limited to malate, oxalate, chloride, bromide, iodide, nitrate, sulfate, bisulfate, phosphate, acid phosphate, isonicotinate, acetate, lactate, salicylate, citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, *p-*toluenesulfonate and pamoate (i.e., 1,1'-methylene-bis-(2-hydroxy-3-naphthoate)) salts. Compounds included in the present compositions that include an amino moiety may form pharmaceutically acceptable salts with various amino acids, in addition to the acids mentioned above. Compounds included in the present compositions that are acidic in nature are capable of forming base salts with various pharmacologically acceptable cations. Examples of such salts include alkali metal or alkaline earth metal salts and, particularly, calcium, magnesium, sodium, lithium, zinc, potassium, and iron salts.

The compounds of the disclosure may contain one or more chiral centers and/or double bonds and, therefore, exist as stereoisomers, such as geometric isomers, enantiomers or diastereomers. The term "stereoisomers" when used herein consist of all geometric isomers, enantiomers or diastereomers. These compounds may be designated by the symbols "R" or "S," depending on the configuration of substituents around the stereogenic carbon atom. The present invention encompasses various stereoisomers of these compounds and mixtures thereof. Stereoisomers include enantiomers and diastereomers. Mixtures of enantiomers or diastereomers may be designated "(±)" in nomenclature, but the skilled artisan will recognize that a structure may denote a chiral center implicitly.

Individual stereoisomers of compounds of the present invention can be prepared synthetically from commercially available starting materials that contain asymmetric or stereogenic centers, or by preparation of racemic mixtures followed by resolution methods well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, or (3) direct separation of the mixture of optical enantiomers on chiral chromatographic columns. Stereoisomeric mixtures can also be resolved into their component stereoisomers by well known methods, such as chiral-phase gas chromatography, chiral-phase high performance liquid chromatography, crystallizing the compound as a chiral salt complex, or crystallizing the compound in a chiral solvent. Stereoisomers can also be obtained from stereomerically-pure intermediates, reagents, and catalysts by well known asymmetric synthetic methods.

Geometric isomers can also exist in the compounds of the present invention. The symbol 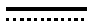 denotes a bond that may be a single, double or triple bond as described herein. The present invention encompasses the various geometric isomers and mixtures thereof resulting from the arrangement of substituents around a carbon-carbon double bond or arrangement of substituents around a carbocyclic ring. Substituents around a carbon-carbon double bond are designated as being in the "*Z*" or "*E*" configuration wherein the terms "*Z*" and "*E"* are used in accordance with IUPAC standards. Unless otherwise specified, structures depicting double bonds encompass both the "*E*" and "*Z*" isomers.

Substituents around a carbon-carbon double bond alternatively can be referred to as "cis" or "trans," where "cis" represents substituents on the same side of the double bond and "trans" represents substituents on opposite sides of the double bond. The arrangement of substituents around a carbocyclic ring are designated as "cis" or "trans." The term "cis" represents substituents on the same side of the plane of the ring and the term "trans" represents substituents on opposite sides of the plane of the ring. Mixtures of compounds wherein the substituents are disposed on both the same and opposite sides of plane of the ring are designated "cis/trans."

The compounds disclosed herein can exist in solvated as well as unsolvated forms with pharmaceutically acceptable solvents such as water, ethanol, and the like, and it is intended that the invention embrace both solvated and unsolvated forms. The compound may be amorphous. The compound may be a polymorph. The compound may be in a crystalline form.

The invention also embraces isotopically labeled compounds which are identical to those recited herein, except that one or more atoms are replaced by an atom having an atomic mass or mass number different from the atomic mass or mass number usually found in nature. Examples of isotopes that can be incorporated into compounds disclosed herein include isotopes of hydrogen, carbon, nitrogen, oxygen, phosphorus, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

Certain isotopically-labeled disclosed compounds (*e.g*., those labeled with ³H and ¹⁴C) are useful in compound and/or substrate tissue distribution assays. Tritiated (*i.e*., ³H) and carbon-14 (*i.e*., ¹⁴C) isotopes are particularly preferred for their ease of preparation and detectability. Further, substitution with heavier isotopes such as deuterium (*i.e*., ²H) may afford certain therapeutic advantages resulting from greater metabolic stability (*e.g*., increased *in vivo* half-life or reduced dosage requirements) and hence may be preferred in some circumstances. Isotopically labeled compounds of the invention can generally be prepared by following procedures analogous to those disclosed in the e.g., Examples herein by substituting an isotopically labeled reagent for a non-isotopically labeled reagent.

The term "prodrug" refers to compounds that are transformed *in vivo* to yield a disclosed compound or a pharmaceutically acceptable salt, hydrate or solvate of the compound. The transformation may occur by various mechanisms, such as through hydrolysis in blood. For example, if a compound disclosed herein or a pharmaceutically acceptable salt, hydrate or solvate of the compound contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

Similarly, if a compound disclosed herein contains an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If a compound disclosed herein incorporates an amine functional group, a prodrug can be formed by the replacement of a hydrogen atom in the amine group with a group such as R-carbonyl, RO-carbonyl, NRR'-carbonyl where R and R' are each independently (C₁-C₁₀)alkyl, (C₃-C₇)cycloalkyl, benzyl, or R-carbonyl is a natural α-aminoacyl or natural α-aminoacyl-natural α-aminoacyl, -C(OH)C(O)OY¹ wherein Y¹ is H, (C₁-C₆)alkyl or benzyl, -C(OY²)Y³ wherein Y² is (C₁-C₄) alkyl and Y³ is (C₁-C₆)alkyl, carboxy(C₁-C₆)alkyl, amino(C₁-C₄)alkyl or mono-N- or diN,N-(C₁-C₆)alkylaminoalkyl, -C(Y⁴)Y⁵ wherein Y⁴ is H or methyl and Y⁵ is mono-N- or diN,N-(C₁-C₆)alkylamino, morpholino, piperidin-1-yl or pyrrolidin-1-yl.

### Compounds

Compounds disclosed for use in one or more of the disclosed methods are represented by formula I, as depicted below. Also disclosed herein are compositions that include a compound represented by formula I and e.g., a pharmaceutically or cosmetically acceptable carrier or excipient. 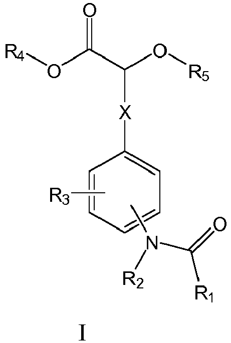
wherein X is C₁-C₃alkylene, optionally substituted with one, two or three substituents selected from halogen or hydroxyl;
R₁ is selected from the group consisting of C₁-C₆alkyl, C₃-C₆cycloalkyl, C₂-C₆alkenyl, and C₂-C₆alkynyl;
R₂ is selected from the group consisting of hydrogen and C₁-C₆alkyl;
R₃ is independently selected, for each occurrence from the group consisting of hydrogen, C₁-C₆alkoxy, C₁-C₆alkyl, cyano, C₃-C₆cycloalkyl, halogen, hydroxyl, and nitro;
R₄ is selected from the group consisting of hydrogen and C₁-C₆alkyl;
R₅ is C₁-C₆alkyl;
or pharmaceutically acceptable salts or N-oxides thereof.

R₁ can be C₁-C₆alkyl, such as methyl. R₂ can be hydrogen. R₃ can be selected from the group consisting of hydrogen, C₁-C₆alkyl, halogen, and hydroxyl. R₃ can be hydrogen. R₄ and R₅ can each be C₁-C₆alkyl. R₄ may be hydrogen and R₅ may be methyl. X may be (CH₂)ₙ, wherein n is 1 or 2, such as 1.

-NR₂-COR₁ can be in the meta position relative to X as shown in formula II.

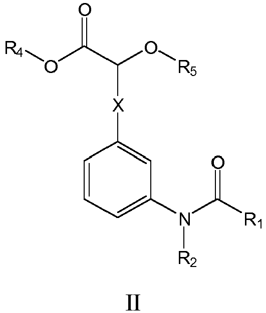

-NR₂-COR₁ can be in the para position relative to X as shown in formula III.

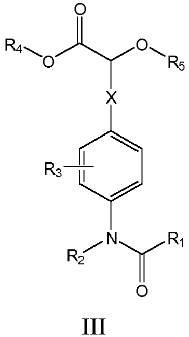

The disclosure provides, at least in part, compounds represented by formula IV, as depicted below. Also disclosed are compositions that include a compound represented by formula IV and e.g., a pharmaceutically acceptable carrier. 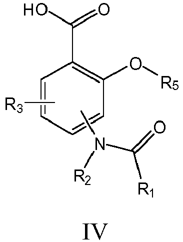
wherein R₁ is selected from the group consisting of C₁-C₆alkyl, C₃-C₆cycloalkyl, C₂-C₆alkenyl, and C₂-C₆alkynyl;
R₂ is selected from the group consisting of hydrogen and C₁-C₆alkyl;
R₃ is independently selected, for each occurrence from the group consisting of hydrogen, C₁-C₆alkoxy, C₁-C₆alkyl, cyano, C₃-C₆cycloalkyl, halogen, hydroxyl, and nitro;
R₅ is hydrogen or C₁-C₆alkyl;
or pharmaceutically acceptable salts or N-oxides thereof.

Compounds of Formula V are also disclosed as shown below, as well as compositions that include a compound represented by formula V and e.g., a pharmaceutically acceptable carrier. 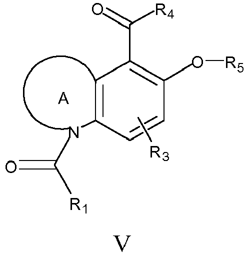
wherein R₁ is selected from the group consisting of C₁-C₆alkyl, C₃-C₆cycloalkyl, C₂-C₆alkenyl, and C₂-C₆alkynyl;
R₃ is independently selected, for each occurrence from the group consisting of hydrogen, C₁-C₆alkoxy, C₁-C₆alkyl, cyano, C₃-C₆cycloalkyl, halogen, hydroxyl, and nitro;
R₄ is selected from the group consisting of hydrogen and C₁-C₆alkyl;
R₅ is hydrogen or C₁-C₆alkyl; and
A is a fused five or six membered heterocycle;
or pharmaceutically acceptable salts or N-oxides thereof.

R₁ can be C₁-C₆alkyl, such as methyl. R₁ and R₃ can each be C₁-C₆alkyl, such as methyl. R₂ can be hydrogen.

Disclosed herein is a compound that can be represented by 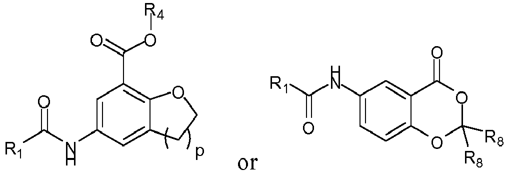 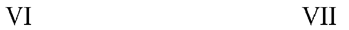
wherein p is 1 or 2;
R₁ is selected from the group consisting of C₁-C₆alkyl, C₃-C₆cycloalkyl, C₂-C₆alkenyl, and C₂-C₆alkynyl;
R₄ and R₈ are each independently selected from the group consisting of hydrogen and C₁-C₆alkyl;
or pharmaceutically acceptable salts or N-oxides thereof.

Compounds, and pharmaceutical compositions, comprising at least one compound, may be selected from the group consisting of: N-acetyl-(R)-(-)-3-(4-aminophenyl)-2-methoxypropionic acid (Compound A), N-acetyl-(S)-(-)-3-(4-aminophenyl)-2-methoxypropionic acid (Compound B), racemic N-acetyl-(S)-(-)-3-(4-aminophenyl)-2-methoxypropionic acid (compound AB); 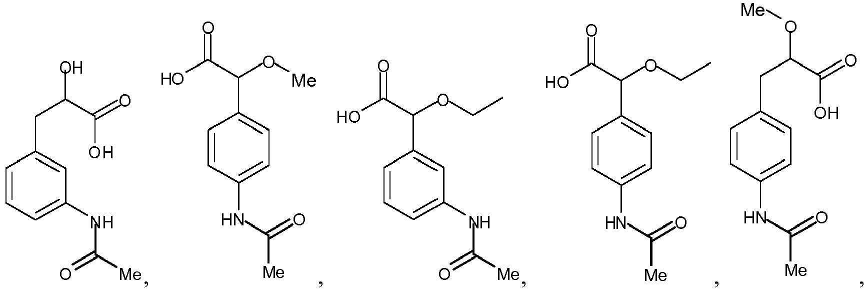 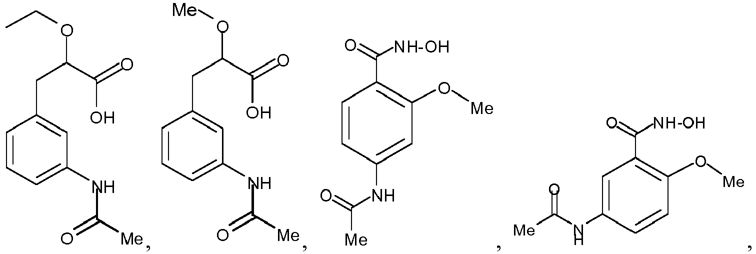 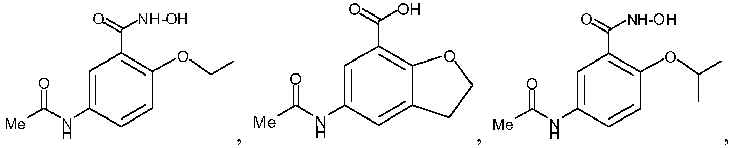 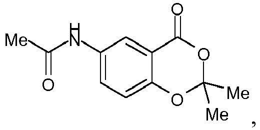 4-acetamino-N-hydroxy-2-methoxybenzamide; 1-acetyl-6-methoxy-1,2,3,4-tetrabydroquinoline-5-carboxylic acid, 5-acetamido-2-hydroxybenzoic acid (e.g., acetylated 5-aminosalicyclic acid) or pharmaceutically acceptable salts or N-oxides thereof. The present disclosure also provides pharmaceutical compositions comprising compounds as disclosed herein formulated together with one or more pharmaceutically or cosmetically acceptable carriers. These formulations include those suitable for oral, rectal, topical, buccal and parenteral (e.g., subcutaneous, intramuscular, intradermal, or intravenous) administration, or for topical use, e.g. as part of a composition suitable for applying topically to skin. Although the most suitable form of administration in any given case will depend on the degree and severity of the condition being treated and on the nature of the particular compound being used.

Additional compounds for use in one or more of the disclosed methods include compounds represented by formula VIII, or a pharmaceutically acceptable salt, enantiomer or stereoisomer thereof: 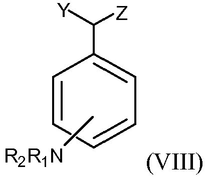 wherein:
R₁ and R₂, are each independently selected from the group consisting of H and C₁₋₆ alkyl; or R₁ and R₂ together with the nitrogen atom they are bonded to form an aromatic or aliphatic ring with 5 or 6 atoms which may be optionally substituted;
Y and Z are each independently selected from the group consisting of H, OH, COOH, -OR₃, -CH(OR₃)COOH; and
R₃ is selected from the group consisting of H, phenyl, benzyl, vinyl, allyl, C₁₋₆ alkyl or C₁₋₆ alkyl substituted by one or more halogens.

Y may be H or COOH. For example, Y may be H and Z may be CH(OR₃)COOH, or Y may be COOH and Z maybe -OR₃. R₃ may be methyl, ethyl, n-propyl, or isopropyl.

The NR₁R₂ moiety may be in the 4' position or may be in the 3' position. R₁ and R₂ are H.

Exemplary compounds also include those represented by formulas IXa or IXb or a pharmaceutically acceptable salt, enantiomer or stereoisomer of: 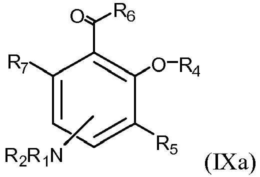 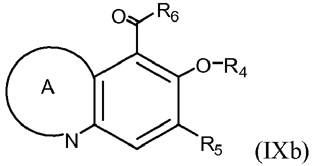 wherein:
R₁ and R₂ are each independently selected from the group consisting of H and C₁₋₆ alkyl; or R₁ and R₂ together, with the nitrogen atom they are bonded to, form an aromatic or aliphatic ring with 5 or 6 atoms;
R₆ is selected from the group consisting of: -NHOH, OH, and -OR₉;
R₉ is C₁₋₆ alkyl;
R₄ is selected from H, phenyl, benzyl, vinyl, allyl, C₁₋₆ alkyl or C₁₋₆ alkyl substituted by one or more halogens;
R₅ and R₇ are each independently hydrogen or halo; or
R₄ and R₅, or R₄ and R₆ together, form a fused heterocyclic ring with 5 or 6 atoms, optionally substituted with halo or C₁₋₆ alkyl; and

A is a fused heterocyclic ring; or a pharmaceutically acceptable salt thereof.

The NR₁R₂ moiety of formula IIa may be in the 4' position or may be in the 3' position. R₁ and R₂ may be H.

R₉ may be methyl, ethyl, n-propyl, or isopropyl.

A compound disclosed herein can be represented by 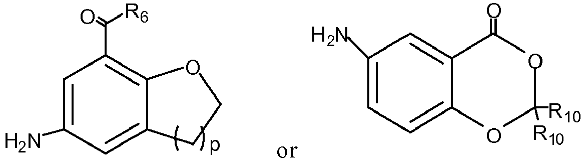 wherein p is 1 or 2, R₆ is OH or -OR₉, wherein R9 is defined above, and R₁₀, independently for each occurrence, is selected from the group consisting of H, halo, or C₁₋₆ alkyl, e.g. methyl or ethyl.

Exemplary compounds contemplated herein include: 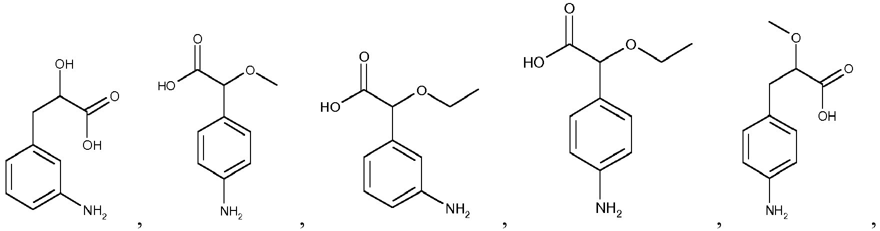 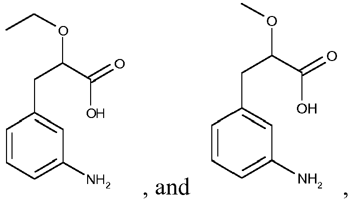 or a pharmaceutically acceptable salt thereof.

Compounds disclosed herein include: 4-amino-N-hydroxy-2-methoxybenzamide (compound 13); 6-methoxy quinoline-5-carboxylic acid (compound 36); 6-methoxy-1,2,3,4-tetrahydroquinoline-5-carboxylic acid (compound 37); 5-diisopropylaminosalicylic acid (compound 38).

Other exemplary compounds include those represented by:

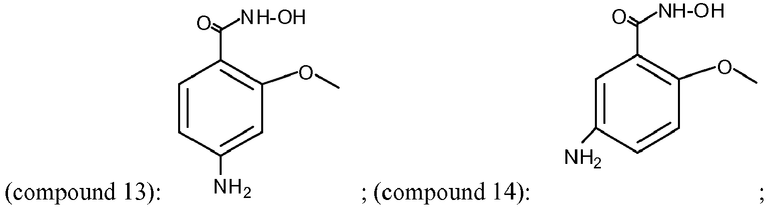

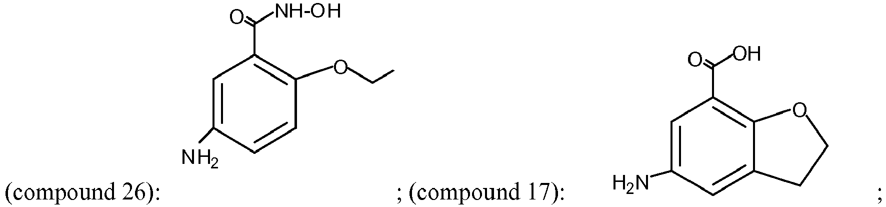

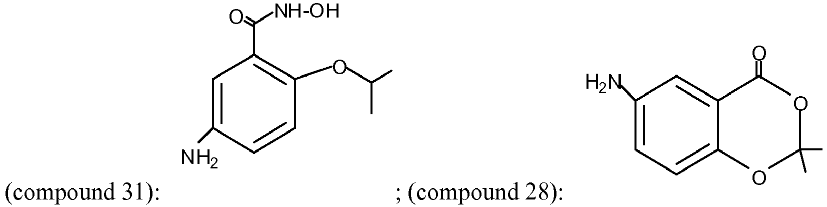

Compounds disclosed herein include racemic mixtures, and enantiomers of compounds, for example: (±)-2-hydroxy-3-(3'-aminophenyl) propionic acid (compound 20); (±)-2-methoxy-2-(4'-aminophenyl) acetic acid (compound 23); (±)-2-ethoxy-2-(3'-aminophenyl) acetic acid (compound 32); (±)-2-ethoxy-2-(4'-aminophenyl) acetic acid (compound 33); (±)-2-methoxy-3-(4'-aminophenyl) propionic acid (compound 34) "±34" (racemic form); (±)-2-ethoxy-3-(4'-aminophenyl) propionic acid (compound 39); (±)-2-ethoxy-3-(3'-aminophenyl) propionic acid (compound 40).

For example, the compounds used in the methods disclosed herein can be enantiomers of the following racemic mixtures: (R,S)-2-hydroxy-2-(3-aminophenyl)acetic acid (compound 10); (R,S)-2-hydroxy-2-(4-aminophenyl)acetic acid (compound 11); (R,S)-2-hydroxy-3-(4'-aminophenyl)propionic acid (compound 21); (R,S)-2-methoxy-2-(3'-aminophenyl)acetic acid (compound 22); (R,S)-2-methoxy-3-(3'-aminophenyl)propionic acid (compound 35); (R,S)-2-methoxy-3-(4-aminophenyl)propionic acid(compound 34), as well as enantiomers, e.g.: (+) 2-S-methoxy-3-(4-aminophenyl)propionic acid(compound 34); (-) 2-R-methoxy-3-(4-aminophenyl)propionic acid (compound 34).

Other racemic type mixtures of compounds disclosed include: e.g. (±)-2-hydroxy-2-(3'-aminophenyl)acetic acid (compound 10); (±)-2-hydroxy-2-(4'-aminophenyl)acetic acid (compound 11); (±)-2-hydroxy-3-(4'-aminophenyl)propionic acid (compound 21) and (±)-2-methoxy-2-(3'-aminophenyl)acetic acid (compound 22).

Further compounds for use in the disclosed methods include: 5-aminosalicylo-hydroxamic acid (compound 5); 3-dimethylaminosalicylic acid (compound 6); 2-methoxy-4-aminobenzoic acid (compound 7); 2-methoxy-5-aminobenzoic acid (compound 8); 5-methylaminosalicylic acid (compound 9); 4-methylaminosalicylic acid (compound 12); 4-acetylaminosalicylic acid (compound 16); 2-ethoxy-4-aminobenzoic acid (compound 18); 2-ethoxy-5-aminobenzoic acid (compound 19); 4-dimethylaminosalicylic acid (compound 24); 2-ethoxy-4-aminobenzoylhydroxamic acid (compound 25); 6-hydroxyquinoline-5-carboxylic acid (compound 27); 2-(2-propyl)oxy-4-aminobenzoic acid (compound 30); 4-(1-piperazinyl)salicylic acid (compound 41); (R,S) 5-oxa-quinoline-6-carboxylic acid (compound 15); 6-methoxy quinoline-5-carboxylic acid (compound 36); 6-methoxy-1,2,3,4-tetrahydroquinoline-5-carboxylic acid (compound 37); 5-diisopropylaminosalicylic acid (compound 38); and 4-diisopropylaminosalicylic acid (compound 42).

The present invention also provides a composition comprising N-acetyl- 3-(4-aminophenyl)-2-methoxypropionic acid or a pharmaceutically acceptable salt or stereoisomer thereof; further comprising at least one compound selected from the group consisting of: pioglitazone, rosiglitazone, doxycycline, hydroxychloroquine, mycophenolate mofetil, rifampin, clindamycin, and spermidine.

Methods for making contemplated compounds may be found for example in WO2007/010516 and WO2007/010514.

### Therapeutic Applications

The disclosure is directed, at least in part, to methods for treating or ameliorating cicatricial alopecia in patient (e.g. a human) comprising administering a therapeutically effective amount of a compound disclosed herein. For example, provided herein are methods for treating or ameliorating cicatricial alopecia comprising topically administering to a patient in need thereof a pharmaceutically acceptable composition comprising N-acetyl-3-(4-aminophenyl)-2-methoxypropionic acid or a pharmaceutically acceptable salt or stereoisomer thereof, and a pharmaceutically acceptable excipient. The cicatricial alopecia is lichen planopilaris. Other cicatricial alopecias include chronic cutaneous lupus erythematosus, keratosis follicularis, spinulosa decalvans, frontal fibrosing alopecia, erythematosus, pseudopelade, central centrifugal alopecia, alopecia mucinosa, keratosis follicularis spinulosadecalvans, folliculitis decalvans, tufted folliculitis, dissecting cellulitis, follicullitis keloidalis, and/or erosive dermatosis.

In certain embodiments, a disclosed composition for use as set out in the claims further comprises, for example, a compound selected from the group consisting of: pioglitazone, rosiglitazone, doxycycline, hydroxychloroquine, mycophenolate mofetil, rifampin, clindamycin, and spermidine.

The disclosure further provides, methods for protecting hair follicle progenitor cells before depilation of mammalian skin using a compound disclosed herein. For example, provided herein are methods for protecting hair follicle progenitor cells before depilation of mammalian skin, comprising contacting the cells with a pharmaceutically acceptable composition comprising N-acetyl-3-(4-aminophenyl)-2-methoxypropionic acid or a pharmaceutically acceptable salt or stereoisomer thereof. The protection of hair follicle progenitor cells allows hair follicles to maintain the ability to regenerate and regrow hair.

The disclosure also provides for methods for enhancing epidermal regeneration in a patient in need thereof using a compound disclosed herein. For example, disclosed herein are methods for enhancing epidermal regeneration in a patient in need thereof (e.g. a patient suffering from a skin wound or burn), comprising topically administering to said patient a pharmaceutically acceptable composition comprising N-acetyl- 3-(4-aminophenyl)-2-methoxypropionic acid or a pharmaceutically acceptable salt or stereoisomer thereof, and a pharmaceutically acceptable excipient.

The disclosure also provides for methods of promoting wound healing in a patent in need thereof using a compound disclosed herein. For example, provided herein are methods of promoting wound healing in a patient, comprising administering to said patient a pharmaceutically acceptable composition comprising N-acetyl-3-(4-aminophenyl)-2-methoxypropionic acid or a pharmaceutically acceptable salt or stereoisomer thereof, and a pharmaceutically acceptable excipient. The wound may be a burn, a scar, a subcutaneous trauma wound, or a surgical wound.

Provided herein is method for protecting and/or preserving a composition comprising hair follicle progenitor cells, wherein the method comprises contacting said hair follicle progenitor cells with N-acetyl-3-(4-aminophenyl)-2-methoxypropionic acid is also provided. Such a protected composition can be used to e.g., to preserve integrity of such cells when used experimentally or as a therapeutic agent (e.g. for hair loss, arthritis, or for repair of damaged tissues). Disclosed herein is a composition comprising N-acetyl-(R)-(-)-3-(4-aminophenyl)-2-methoxypropionic acid and hair follicle progenitor cells.

A disclosed composition for use as set out in the claims comprises N-acetyl-(R)-(-)-3-(4-aminophenyl)-2-methoxypropionic acid. In other embodiments the disclosed composition for use comprises a concentration of at least about 1 mM of the N-acetyl-3-(4-aminophenyl)-2-methoxypropionic acid. For example, a disclosed composition may comprise a concentration of at least about 1 mM, about 2 mM, about 3 mM, about 4 mM, about 5 mM, about 6 mM, about 7 mM, about 8 mM, about 9 mM or about 10 mM. In certain other embodiments, a disclosed composition comprises a concentration of at least about 1 mM to about 10 mM of N-acetyl- 3-(4-aminophenyl)-2-methoxypropionic acid. For example, a disclosed compositions may comprise a concentration of at least about 1.5 mM to about 9.5 mM, about 2 mM to about 9 mM, about 2.5 mM to about 8.5 mM, about 3 mM to about 8 mM, about 3.5 mM to about 7.5 mM, about 4 mM to about 7 mM or about 4.5 mM to about 6.5 mM.

For example, disclosed methods may include topically applying a composition having a high concentration of a disclosed compound, e.g., that provides an effective amount significantly greater than the amount necessary to, e.g., induce hair growth. For example, such high concentration of such a composition may include about 1mM to about 1000mM or more of a disclosed compound, e.g. about 1mM to about 100mM, about 10mM to about 100mM, or about 10mM to about 50mM.

The compounds disclosed herein may be administered to subjects (animals and/or humans) in need of such treatment in dosages that will provide optimal pharmaceutical efficacy. It will be appreciated that the dose required for use in any particular application will vary from patient to patient, not only with the particular compound or composition selected, but also with the route of administration, the nature of the condition being treated, the age and condition of the patient, concurrent medication or special diets then being followed by the patient, and other factors which those skilled in the art will recognize, with the appropriate dosage ultimately being at the discretion of the attendant physician. For treating clinical conditions and diseases noted above, a disclosed compound or composition may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques.

Dosing frequency can vary, depending on factors such as route of administration, dosage amount and the disease condition being treated. Exemplary dosing frequencies are at least once per day, at least once per week and at least once every two weeks.

Contemplated formulations or compositions comprise a disclosed compound and typically may also include a pharmaceutically acceptable carrier or expicient.

Contemplated compositions may be administered by various means, depending on their intended use, as is well known in the art. Compositions of the invention are for topical administration. These formulations may be prepared by conventional means, and, if desired, disclosed compositions may be mixed with any conventional additive, such as an excipient, a binder, a disintegrating agent, a lubricant, a corrigent, a solubilizing agent, a suspension aid, an emulsifying agent or a coating agent.

In formulations disclosed herein, wetting agents, emulsifiers and lubricants, such as sodium lauryl sulfate and magnesium stearate, as well as coloring agents, release agents, coating agents, sweetening, flavoring and perfuming agents, preservatives and antioxidants may be present in the formulated agents.

Methods of preparing these formulations include the step of bringing into association compositions disclosed herein with the carrier and, optionally, one or more accessory ingredients. In general, the formulations are prepared by uniformly and intimately bringing into association agents with liquid carriers, or finely divided solid carriers, or both, and then, if necessary, shaping the product.

Suspensions, in addition to the subject composition, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Dosage forms for transdermal or topical administration of a subject composition include powders, sprays, ointments, pastes, creams, lotions, gels, solutions, patches and inhalants. The active component may be mixed under sterile conditions with a pharmaceutically acceptable carrier, and with any preservatives, buffers, or propellants which may be required.

The ointments, pastes, creams and gels may contain, in addition to a subject composition, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays may contain, in addition to a subject composition, excipients such as lactose, talc, silicic acid, aluminum hydroxide, calcium silicates and polyamide powder, or mixtures of these substances. Sprays may additionally contain customary propellants, such as chlorofluorohydrocarbons and volatile unsubstituted hydrocarbons, such as butane and propane.

Compositions and compounds of the present disclosure may alternatively be administered by aerosol. This is accomplished by preparing an aqueous aerosol, liposomal preparation or solid particles containing the compound. A non-aqueous (e.g., fluorocarbon propellant) suspension could be used. Sonic nebulizers may be used because they minimize exposing the agent to shear, which may result in degradation of the compounds contained in the subject compositions.

Ordinarily, an aqueous aerosol is made by formulating an aqueous solution or suspension of a subject composition together with conventional pharmaceutically acceptable carriers and stabilizers. The carriers and stabilizers vary with the requirements of the particular subject composition, but typically include non-ionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Pharmaceutical compositions disclosed herein may be suitable for parenteral administration comprise a subject composition in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions disclosed herein include water, ethanol, polyols (such as glycerol, propylene glycol, polyethylene glycol, and the like), and suitable mixtures thereof, vegetable oils, such as olive oil, and injectable organic esters, such as ethyl oleate and cyclodextrins. Proper fluidity may be maintained, for example, by the use of coating materials, such as lecithin, by the maintenance of the required particle size in the case of dispersions, and by the use of surfactants. The efficacy of treatment with the subject compositions may be determined in a number of fashions known to those of skill in the art.

Throughout the description, where compositions are described as having, including, or comprising specific components, it is contemplated that compositions also consist essentially of, or consist of, the recited components. Similarly, where processes are described as having, including, or comprising specific process steps, the processes also consist essentially of, or consist of, the recited processing steps. Except where indicated otherwise, the order of steps or order for performing certain actions are immaterial so long as the invention remains operable. Moreover, unless otherwise noted, two or more steps or actions may be conducted simultaneously.

### EXAMPLES

The compounds disclosed herein can be prepared in a number of ways well known to one skilled in the art of organic synthesis. More specifically, compounds disclosed herein may be prepared using the reactions and techniques described herein. In the description of the synthetic methods described below, it is to be understood that all proposed reaction conditions, including choice of solvent, reaction atmosphere, reaction temperature, duration of the experiment and workup procedures, can be chosen to be the conditions standard for that reaction, unless otherwise indicated. It is understood by one skilled in the art of organic synthesis that the functionality present on various portions of the molecule should be compatible with the reagents and reactions proposed. Substituents not compatible with the reaction conditions will be apparent to one skilled in the art, and alternate methods are therefore indicated. The starting materials for the examples are either commercially available or are readily prepared by standard methods from known materials.

### Example 1 Preparation of N-acetyl-(R)-(-)-3-(4-aminophenyl)-2-methoxypropionic acid (N-Acetyl E2); Compound A

To (R)-(-)-3-(4-aminophenyl)-2-methoxypropionic acid (40 g) in a 0.5L glass reactor was added ethyl acetate (80 g) and acetic anhydride (62.8 g). The mixture was stirred at 90 °C for 1 hour. Upon cooling, the solvent was removed by vaccum distillation, providing an oily residue. To this residue was added water (120 g) and ethyl acetate (120 g). After stirring for 10 min at 35 °C, the layers were separated and the aqueous layer discarded. The organic layer solvent was removed by vacuum distillation. Acetone (120 g) was then added and the resulting mixture was warmed until dissolution was complete. The solution was cooled to 0 °C, and the product precipitated which was collected by filtration. The solid was rinsed with acetone (20 g) and dried at 65 °C to afford 26g of the title compound.

### Example 2 Effects of Compound A on stem cell markers

The aim of the present study is to determine the effects of Compound A on stem cell markers in the hair follicle by evaluating the expression of stem call markers K15 and K19.

### Materials and methods

### Tissue specimens

Normal human scalp skin was obtained from 6 women undergoing routine face-lift surgery after informed consent. All experiments were performed according to Helsinki guidelines, with appropriate ethics committee approval. Details on specimen origin are listed in Table 1.

**Table 1. Specimen details.**

| | **Age** | **Scalp region** |
|---|---|---|
| **Patient 1** | 48 | Occipital scalp |
| **Patient 2** | 56 | Occipital scalp |
| **Patient 3** | 59 | Occipital scalp |
| **Patient 4** | 55 | Occipital scalp |
| **Patient 5** | 68 | Occipital scalp |
| **Patient 6** | 67 | Occipital scalp |

### Hair follicle microdissection and organ culture

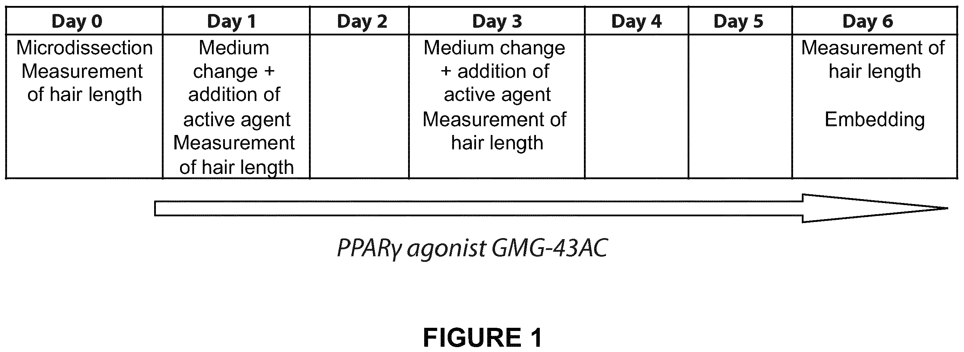

Normally pigmented anagen VI hair follicles (HFs) (grey/white HFs were excluded from the study) were microdissected from normal human scalp skin and organ cultured following the Philpott model. Compound A or vehicle was administered once for each change of medium (i.e. every 48h/72h). An overview of the experimental procedures is presented in Figure 1.

### K15 quantitative immunohistochemistry

To investigate keratin K15 expression, the tyramide signal amplification method was used as previously described (Kloepper et al., 2008). Briefly, acetone fixed cryosections were washed three times for 5 min using TNT (Tris-HCL NaCl Tween) buffer (0.1 mol/l Tris-HCl, pH 7.5; containing 0.15 mol/l NaCl and 0.05% Tween 20). Next, horseradish peroxidase was blocked by washing with 3% H₂O₂ in phosphate-buffered saline (PBS) for 15 min. Preincubation was performed with the incubation of avidin and biotin for 15 min and 5% goat normal serum in TNT for 30 min with washing steps in between. Mouse anti-human K15 (clone LHK15, Chemicon, Billerica, USA) was diluted in TNT and incubated overnight at 4°C followed by a biotinylated secondary antibody goat anti-mouse (1:200 in TNT) for 45 min at RT. Next, streptavidin horseradish peroxidase (TSA kit; Perkin-Elmer, Boston, MA, USA) was administered (1:100 in TNT) for 30 min at RT. The reaction was amplified by FITC-tyramide amplification reagent at RT for 5 min (1:50 in amplification diluent provided with the kit). The intensity of the immunostaining was quantified by ImageJ software (National Institutes of Health). The staining intensity of defined reference regions in the HF was measured and compared between control and N Compound A-treated groups. The percentage of K15-positive cells in comparison to the total amount of cells in the ORS was calculated.

### K19 quantitative immunohistochemistry

A previously described protocol was used to investigate K19 expression (Kloepper *et al*., 2008). Briefly, acetone-fixed cryosections were pre-treated with goat serum (10% in Tris-buffered saline, Dako). The sections were incubated first with primary antibodies against K19 (mouse anti-human: K19 - 1:10; overnight, at 4°C; PROGEN, Heidelberg, Germany;) and then with FITC-labeled goat anti-mouse (1:200 in TBS, for 45 min, RT, Jackson ImmunoResearch) immunoglobulins as secondary antibodies. Counterstaining was performed with DAPI (Boehringer Mannheim, Mannheim, Germany). The intensity of this immunostaining was quantified by ImageJ software (National Institutes of Health). The staining intensity of defined reference regions in the HF was measured and compared between control and Compound A-treated groups. The percentage of K19-positive cells in comparison to the total amount of cells in the ORS was calculated.

Statistical analysis was performed using a two-tailed Student's *t-test* for unpaired samples. For meta-analysis purposes, a total of six assays (each with HFs from a different female individual) were run. For keratin 19, only 5 assays were available for analysis since the number of usable hair follicle sections for quantitative immunohistomorphometry did not suffice to also run this parameter. In order to avoid data distortion by individual experiments, rigid exclusion criteria were defined that allowed to exclude one individual experiment (out of 5-6) per read-out parameter. Since these exclusion criteria differed for each study parameter, different experiments (i.e. one out of 6, and one out of 5 in the case of keratin 19 analysis) were excluded for each assay parameter. The exclusion criteria were: (i) most extreme deviation from the results trend shown by the majority among the 6 experiments, in order to avoid data distortion by outliers (which can be affected e.g. by patient's medication, medical history etc.); and (ii) failure to meet minimal quality criteria.

### Results

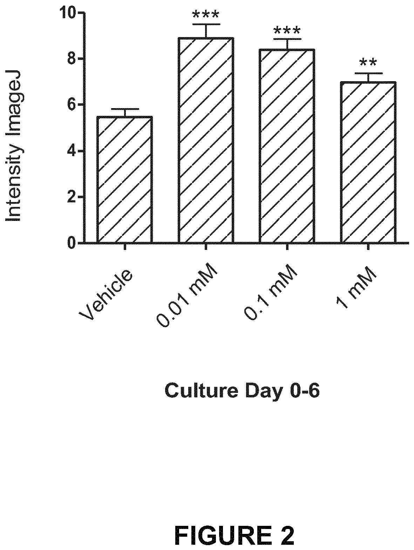

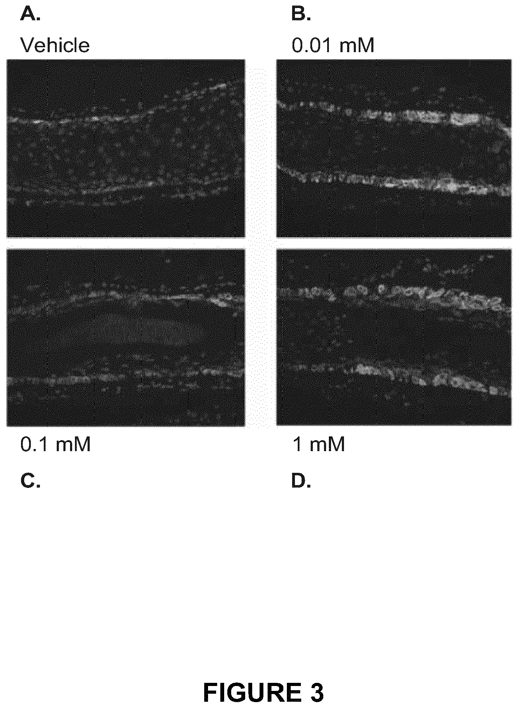

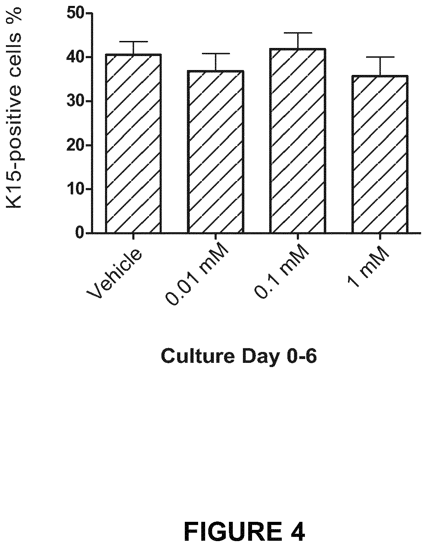

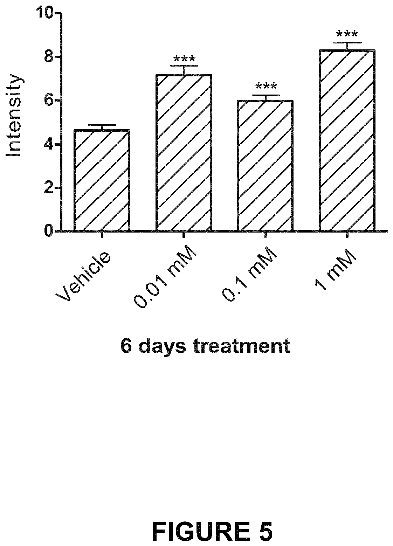

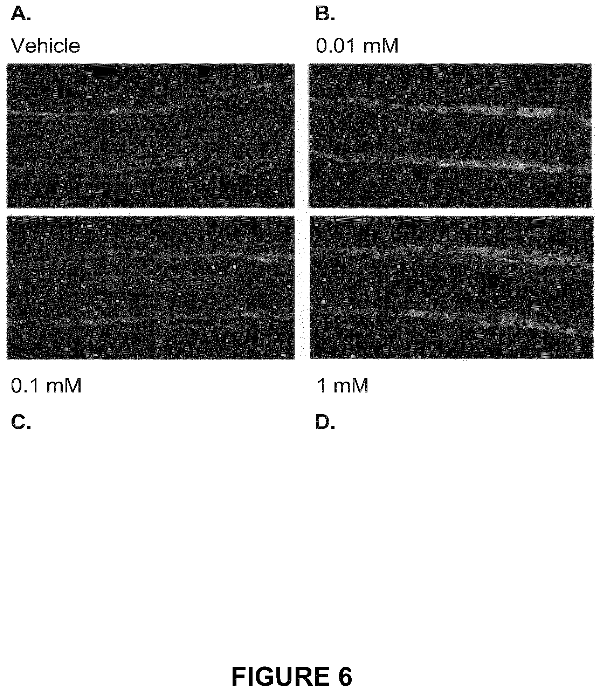

Administration of Compound A strongly stimulated Keratin -15 immunoreactivity at all concentrations tested (Figures 2 and 3), although the number of K15-positive cells did not increase (Figure 4). Administration of Compound A also strongly stimulated Keratin 19 immunoreactivity at all concentrations tested (Figure 5), and also had a stimulating effect on the number of K19-positive cells relative to control (Figure 6). The data indicate that Compound A possesses hair follicle progenitor/stem cell-"protective" properties.

### Example 3 Effect of Compound A on Hair Shaft Elongation

The aim of the present study is to determine the effect of Compound A on hair shaft elongation.

### Materials and methods

### Tissue Specimens

Details regarding tissue specimen origin used in the present example are as described in Example 2 above.

### LDH measurement

LDH activity in the supernatant can serve as an indicator of cytotoxicity and was measured following the manufacturer's instructions (Cytotoxicity Detection Kit; Roche, Mannheim, Germany). The absorbance of the samples was measured at 490 nm using an ELISA plate-reader.

### Hair shaft elongation

Hair shaft length measurements of HFs were performed on individual HFs using a Zeiss inverted binocular microscope with an eyepiece measuring graticule.

### HF cycle staging

HF cycle staging was carried out according to previously defined morphological criteria, and the percentage of HFs in anagen and early, mid, or late catagen was determined.

### Proliferation and apoptosis measurements

To evaluate apoptotic cells in colocalization with a proliferation marker Ki-67, a Ki-67/terminal dUTP nick-end labeling (TUNEL) double-staining method was used. Cryostat sections were fixed in paraformaldehyde and ethanol-acetic acid (2:1) and labeled with a digoxigenin-deoxy-UTP (ApopTag fluorescein *in situ* apoptosis detection kit; Intergen, Purchase, NY) in the presence of terminal deoxynucleotidyl transferase, followed by incubation with a mouse anti-Ki-67 antiserum (1:20 in PBS overnight at 4 C; Dako, Glostrup, Denmark). TUNEL-positive cells were visualized by an antidigoxigenin fluorescein isothiocyanate-conjugated antibody (ApopTag kit), whereas Ki-67 was detected by a rhodamine-labeled goat antimouse antibody (Jackson ImmunoResearch, West Grove, PA). Negative controls were performed by omitting terminal deoxynucleotidyl transferase and the Ki-67 antibody. Counterstaining was performed with 4',6-diamidino-2-phenylindole (DAPI) (Roche Molecular Biochemicals GmbH, Mannheim, Germany). Quantitative immunohistomorphometry was performed; Ki-67-, TUNEL-, or DAPI-positive cells were counted in a previously defined reference region of the HF matrix, and the percentage of Ki-67/TUNEL-positive cells were determined. Statistical analysis was performed using a two-tailed Student's *t-test* for unpaired samples. For meta-analysis purposes, a total of six assays (each with HFs from a different female individual) were run. In order to avoid data distortion by individual experiments, rigid exclusion criteria were defined that allowed to exclude one individual experiment (out of 5-6) per read-out parameter. Since these exclusion criteria differed for each study parameter, different experiments (i.e. one out of 6) were excluded for each assay parameter. The exclusion criteria were: (i)most extreme deviation from the results trend shown by the majority among the 6 experiments, in order to avoid data distortion by outliers (which can be affected e.g. by patient's medication, medical history etc.); and (ii) failure to meet minimal quality criteria.

### Results

### LDH activity

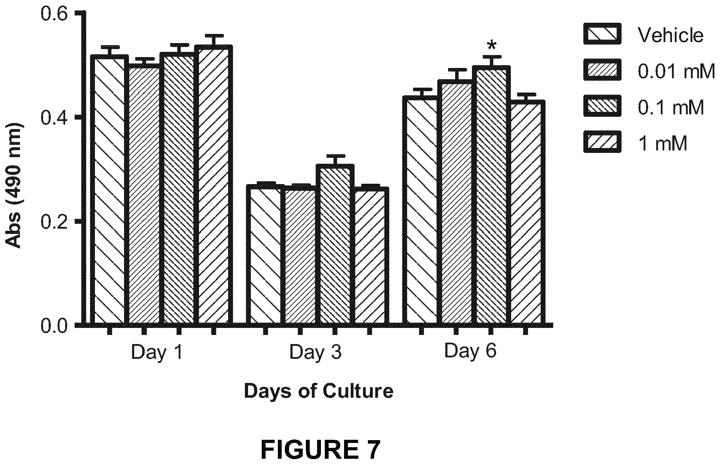

Measurement of LDH activity in the supernatant (parameter of cell death and cell lysis) showed a slight increase in LDH activity, only in the 0.1 mM dose on day 6 (Figure 7). This suggests very low, if any, Compound A-associated HF toxicity under assay conditions.

### Hair shaft elongation

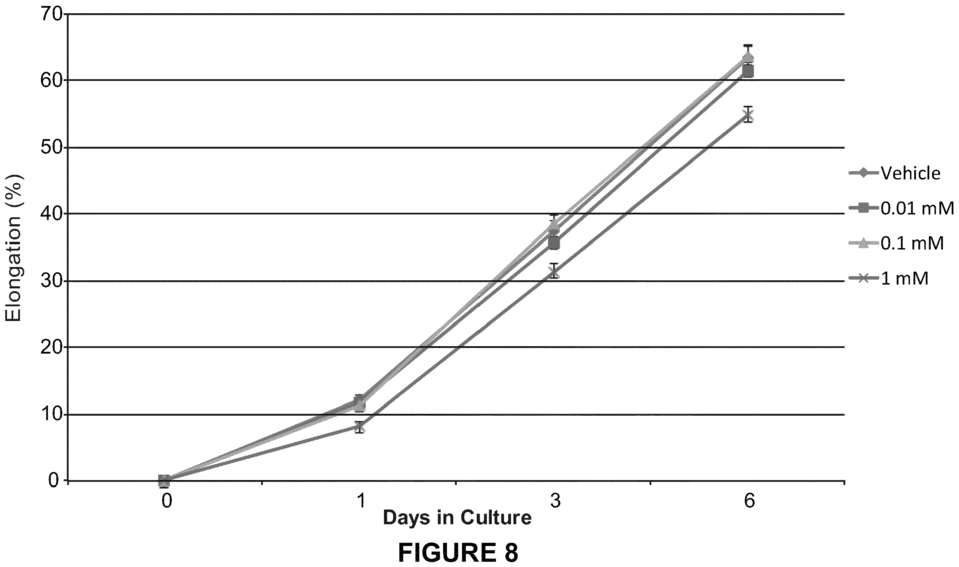

Administration of Compound A slightly, but significantly, inhibited hair shaft elongation in the high dose (1 mM) (Figure 8). The lower concentrations did not have an effect on elongation.

### Hair cycle effects

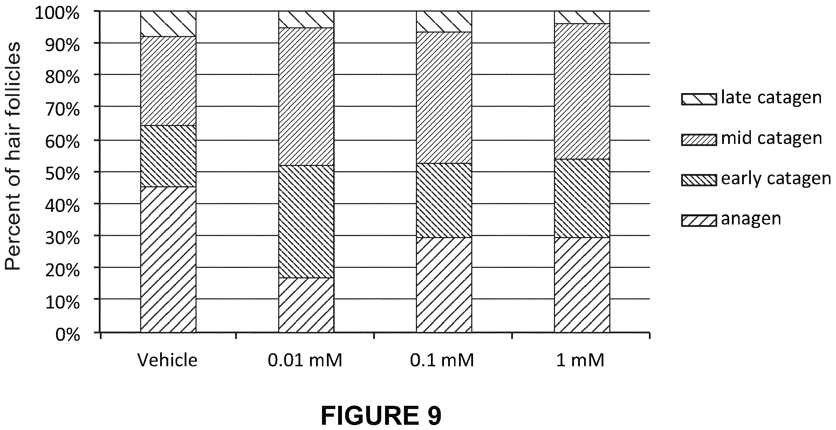

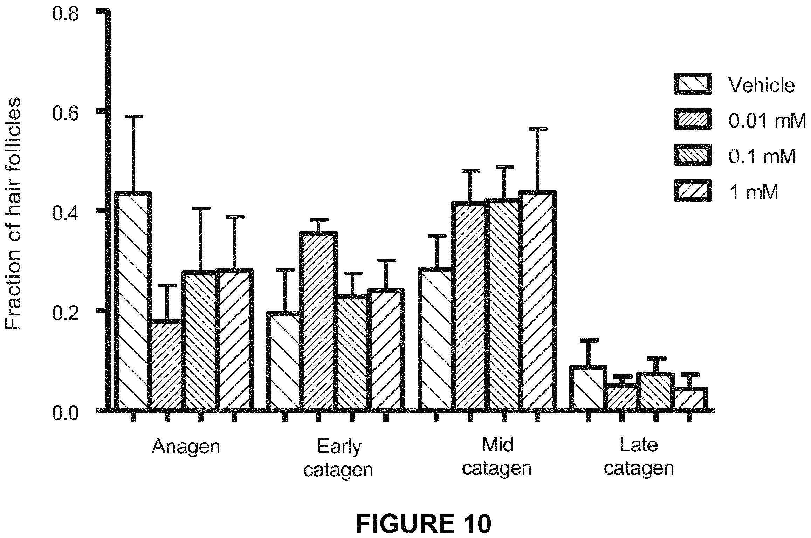

In general, Compound A induced catagen at all concentrations, in agreement with the reduced hair shaft elongation seen with the high dose (1 mM) (Figure 9). A strong trend towards induction of catagen was also evident in analysis of the individual 5 experiments (Figure 10).

The data indicate that high doses of Compound A (e.g., 1 mM or greater) inhibits hair shaft elongation in occipital human female scalp hair shafts. In addition, all tested concentrations of Compound A have a catagen-inducing effect, which may underlie the decreased hair shaft elongation observed at the high dose.

### References

All publications and patents mentioned herein, including those items listed below, are hereby incorporated by reference in their entirety as if each individual publication or patent was specifically and individually incorporated by reference. In case of conflict, the present application, including any definitions herein, will control.

### Equivalents

While specific embodiments of the subject invention have been discussed, the above specification is illustrative and not restrictive. Many variations of the invention will become apparent to those skilled in the art upon review of this specification. The full scope of the invention should be determined by reference to the claims.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in this specification and attached claims are approximations that may vary depending upon the desired properties sought to be obtained by the present invention.

The words "comprises/comprising" and the words "having/including" when used herein with reference to the present invention are used to specify the presence of stated features, integers, steps or components but do not preclude the presence or addition of one or more other features, integers, steps, components or groups thereof.

## Claims

1. A pharmaceutical composition comprising N-acetyl-3-(4'-aminophenyl)-2-methoxypropionic acid, or a pharmaceutically acceptable salt or a stereoisomer thereof, and a pharmaceutically acceptable excipient, for use in treating or ameliorating lichen planopilaris in a patient in need thereof, wherein the composition is for topical administration to said patient.

2. The composition for use according to claim 1, further comprising a compound selected from the group consisting of pioglitazone, rosiglitazone, doxycycline, hydroxychloroquine, mycophenolate mofetil, rifampin, clindamycin, and spermidine.

3. The composition for use according to claim 1 or 2, wherein the patient is a human.

4. The composition for use according to any one of claims 1-3, wherein the stereoisomer of N-acetyl-3-(4'-aminophenyl)-2-methoxypropionic acid is N-acetyl-(R)-3-(4'-aminophenyl)-2-methoxypropionic acid, or a pharmaceutically acceptable salt thereof.

5. The composition for use according to any one of claims 1-3, wherein the stereoisomer of N-acetyl-3-(4'-aminophenyl)-2-methoxypropionic acid is N-acetyl-(S)-3-(4'-aminophenyl)-2-methoxypropionic acid, or a pharmaceutically acceptable salt thereof.

6. The composition for use according to any one of claims 1-5, wherein the composition comprises a concentration of at least about 1 mM of N-acetyl-3-(4'-aminophenyl)-2-methoxypropionic acid, or a pharmaceutically acceptable salt or a stereoisomer thereof.

7. The composition for use according to any one of claims 1-6, wherein the composition comprises a concentration of about 1 mM to about 10 mM of N-acetyl-3-(4'-aminophenyl)-2-methoxypropionic acid, or a pharmaceutically acceptable salt or a stereoisomer thereof.

## Patentansprüche

1. Pharmazeutische Zusammensetzung, welche N-Acetyl-3-(4'-aminophenyl)-2-Methoxy-propionsäure oder ein pharmazeutisch zulässiges Salz oder Stereoisomer davon und einen pharmazeutisch zulässigen Hilfsstoff aufweist zur Verwendung bei der Behandlung oder zur Besserung von Lichen planopilaris bei einem Patienten, der davon betroffen ist, wobei die Zusammensetzung zur lokalen Anwendung bei dem Patienten vorgesehen ist.

2. Zusammensetzung zur Verwendung nach Anspruch 1, welche ferner eine Verbindung aufweist, die aus der Gruppe ausgewählt ist, die aus Pioglitazon, Rosiglitazon, Doxycyclin, Hydroxychloroquin, Mycophenolat-Mofetil, Rifampicin, Clindamycin und Spermidin besteht.

3. Zusammensetzung zur Verwendung nach Anspruch 1 oder 2, wobei der Patient ein Mensch ist.

4. Zusammensetzung zur Verwendung nach einem der Ansprüche 1 - 3, wobei das Stereoisomer von N-Acetyl-3-(4'-aminophenyl)-2-Methoxy-propionsäure N-Acetyl-(R)-3-(4'-aminophenyl)-2-Methoxy-propionsäure oder ein pharmazeutisch zulässiges Salz davon ist.

5. Zusammensetzung zur Verwendung nach einem der Ansprüche 1 - 3, wobei das Stereoisomer von N-Acetyl-3-(4'-aminophenyl)-2-Methoxy-propionsäure N-Acetyl-(S)-3-(4'-aminophenyl)-2-Methoxy-propionsäure oder ein pharmazeutisch zulässiges Salz davon ist.

6. Zusammensetzung zur Verwendung nach einem der Ansprüche 1 - 5, wobei die Zusammensetzung eine Konzentration von mindestens ungefähr 1 mM N-Acetyl-3-(4'-aminophenyl)-2-Methoxy-propionsäure oder eines pharmazeutisch zulässigen Salzes oder eines Stereoisomers davon aufweist.

7. Zusammensetzung zur Verwendung nach einem der Ansprüche 1 - 6, wobei die Zusammensetzung eine Konzentration von ungefähr 1 mM bis ungefähr 10 mM N-Acetyl-3-(4'-aminophenyl)-2-Methoxy-propionsäure oder eines pharmazeutisch zulässigen Salzes oder eines Stereoisomers davon aufweist.

## Revendications

1. Composition pharmaceutique comprenant de l'acide N-acétyl-3-(4'-aminophényl)-2-méthoxypropionique, ou un sel ou un stéréoisomère de celui-ci acceptable sur le plan pharmaceutique, et un excipient acceptable sur le plan pharmaceutique, pour utilisation dans le traitement ou l'amélioration du lichen planopilaris sur un patient en ayant besoin, la composition étant destinée à une administration topique au patient.

2. Composition pour une utilisation selon la revendication 1, comprenant en outre un composé sélectionné dans le groupe comprenant la pioglitazone, la rosiglitazone, la doxycycline, l'hydroxychloroquine, le mycophénolate mofétil, la rifampicine, la clindamycine et la spermidine.

3. Composition pour utilisation selon la revendication 1 ou 2, dans laquelle le patient est un humain.

4. Composition pour utilisation selon l'une quelconque des revendications 1 à 3, dans laquelle le stéréoisomère de l'acide N-acétyl-3-(4'-aminophényl)-2-méthoxypropionique est l'acide N-acétyl-(R)-3-(4'-aminophényl)-2-méthoxypropionique, ou un sel de celui-ci acceptable sur le plan pharmaceutique.

5. Composition pour utilisation selon l'une quelconque des revendications 1 à 3, dans laquelle le stéréoisomère de l'acide N-acétyl-3-(4'-aminophényl)-2-méthoxypropionique est l'acide N-acétyl-(S)-3-(4'-aminophényl)-2-méthoxypropionique, ou un sel de celui-ci acceptable sur le plan pharmaceutique.

6. Composition pour utilisation selon l'une quelconque des revendications 1 à 5, dans laquelle la composition comprend une concentration d'au moins environ 1 mM d'acide N-acétyl-3-(4'-aminophényl)-2-méthoxypropionique, ou d'un sel ou d'un stéréoisomère de celui-ci acceptable sur le plan pharmaceutique.

7. Composition pour utilisation selon l'une quelconque des revendications 1 à 6, dans laquelle la composition comprend une concentration d'environ 1 mM à environ 10 mM d'acide N-acétyl-3-(4'-aminophényl)-2-méthoxypropionique, ou d'un sel ou d'un stéréoisomère de celui-ci acceptable sur le plan pharmaceutique.
